Europäisches Patentamt

European Patent Office

Office européen des brevets

(11) Publication number: **0 130 619**
**B1**

(12) **EUROPEAN PATENT SPECIFICATION**

(45) Date of publication of patent specification: **15.03.89**

(51) Int. Cl.⁴: **A 61 K 39/29**

(21) Application number: **84107773.8**

(22) Date of filing: **04.07.84**

(54) **Hepatitis B vaccine and method for its preparation.**

(30) Priority: **05.07.83 JP 122945/83**

(43) Date of publication of application:
**09.01.85 Bulletin 85/02**

(45) Publication of the grant of the patent:
**15.03.89 Bulletin 89/11**

(84) Designated Contracting States:
**AT BE CH DE FR GB IT LI NL SE**

(56) References cited:
**FR-A-1 588 829**
**GB-A-1 442 564**
**US-A-2 879 202**
**US-A-3 636 191**
**US-A-3 674 864**

(73) Proprietor: **Juridical Foundation The Chemo-
Sero-Therapeutic Research Institute
668, Okubo Shimizu-machi
Kumamoto-shi Kumamoto-ken (JP)**

(72) Inventor: **Mizuno, Kyosuke
1725-1, Kamitatsuta Tatsuta-machi
Kumamoto-shi Kumamoto-ken (JP)**

Inventor: **Ishihara, Yoshimitsu
297-6, Shimonabe-machi
Kumamoto-shi Kumamoto-ken (JP)**

Inventor: **Kawahara, Tetsuo
1151, Muro Ozumachi
Kikuchi-gun Kumamoto-ken (JP)**

Inventor: **Ohtomo, Nobuya
6-22-29, Shimasaki-machi
Kumamoto-shi Kumamoto-ken (JP)**

(74) Representative: **Vossius & Partner
Siebertstrasse 4 P.O. Box 86 07 67
D-8000 München 86 (DE)**

Courier Press, Leamington Spa, England.

# EP 0 130 619 B1

**Description**

The present invention relates to a lyophilized preparation of a hepatitis B vaccine. More particularly, it relates to an improved lyophilized preparation of a hepatitis B vaccine which is obtained by lyophilizing an inactivated purified hepatitis B virus surface antigen (HBsAg) in the presence of an aluminum gel and a particular combination of stabilizers.

From US—A—3 636 191 a vaccine against viral hepatitis is known consisting of Australia antigen particles and an adjuvant such as aluminum hydroxide or aluminum phosphate.

From US—A—3 674 864 a process for the preparation of inactivated virus materials which have increased stability of surface structures and increased interfering power during freezing or freeze-drying is known. In this process an aqueous solution containing 0.5 to 25 per cent by weight of a dissaccharide, monosaccharide or sugar alcohol is used as stabilizer.

From GB—A—1 442 564 a process for preparing an inactivated hepatitis B antigen (HBAg) vaccine which comprises heating a solution in an aqueous medium of a human plasma protein containing HBAg at a temperature from 60 to 120°C for a period of time sufficient to remove the infectivity of the HBAg without the HBAg losing its antigenicity in the presence of at least 5% (W/V) of monosaccharide, disaccharide or sugar alcohol or a mixture thereof as stabilizer.

From US—A—2 879 202 a method of stabilizing live avirulent virus particles is known. In this method 0.5 to 60 percent by weight of sorbitol, mannitol, inositol or dulcitol are added to the virus.

From FR—A—1˙588 829 a method of inactivating infectious microorganisms and/or their antigens in particular rabies virus is known which comprises adding a stabilizer to an aqueous suspension of this material and lyophilizing or heating the mixture at a temperature of 30 to 110°C for 15 minutes to 12 weeks. Examples of stabilizers are degraded gelatin, saccharose, saccharose and glutamate, maltose and glutamate or glucose and gelatin.

Hepatitis B vaccine is usually prepared by using a highly purified HBsAg which is isolated from HBsAg positive human serum, and hence, it is very valuable. It is essential to provide a preparation which is stable and can be kept for a long period of time.

Hepatitis B vaccine preparations usually contain an aluminum gel. In order to prepare a lyophilized preparation of hepatitis B vaccine, first HBsAg must be dissolved in a saline solution for injection or distilled water for injection and then the solution is mixed with the aluminum gel. In such a method, the adsorption of HBsAg onto the aluminum gel varies depending on the order of steps for the preparation, temperature, conditions for shake-mixing, etc., hence the immunogenic activity may vary.

When a conventional liquid preparation of HBsAg is lyophilized the antigen titer is lowered during the drying step. Thus, it is necessary to lyophilize HBsAG in the presence of a stabilizer. It was not known under what conditions the lyophilization of HBsAG can be done in the presence of an aluminum gel and further how the lyophilization conditions affect the stability of the preparation.

Thus, the technical problem underlying the present invention is to provide an improved lyophilized preparation of hepatitis B vaccine having a high antigen titer and excellent storage stability. This problem is solved by the present invention. The invention relates to the subject matter of the claims.

The starting purified HBsAg can be prepared from HBsAg positive human serum by conventional purification methods, such as ultracentrifugation, iron exchange chromatography, gel filtration chromatography, or the like. When the purified HBsAg is used as a vaccine, it is usually inactivated, for example, by subjecting it to heat treatment at 60°C for 10 hours, or to treatment with 0.05 V/V % formalin at 37°C for 96 hours. The inactivated, purified HBsAg is adjusted to a protein content of not more than 0.1 W/V %, preferably not more than 0.02 W/V %, usually 0.005 to 0.02 W/V %.

For the adsorption of HBsAg onto aluminum gel the HBsAg and the aluminum gel are used in a weight ratio of 1:3 to 1:10. The adsorption is carried out by mixing an HBsAg-containing solution with an aluminum gel-containing solution, or by adding an aluminum chloride-containing solution to an HBsAg-containing solution, and adding thereto an aqueous sodium hydroxide solution having an appropriate concentration, by which aluminum hydroxide gel is produced and simultaneously HBsAg is adsorbed thereto. When an aqueous trisodium phosphate solution is used instead of the aqueous sodium hydroxide solution, aluminum phsophate gel is produced and HBsAg is adsorbed thereto. Thus, the aluminum gel used in the present invention includes aluminum hydroxide gel and aluminum phosphate gel. The HBsAg-containing solution is usually an aqueous solution of a neutral salt, such as physiological saline solution. The neutral salt includes sodium chloride, potassium chloride, magnesium chloride. The preferred neutral salt is sodium chloride, which may be used as a mixture with other neutral salts as mentioned above. The neutral salts are usually present in a concentration of 0.1 to 3 W/V %, preferably 0.5 to 2 W/V %.

The HBsAg-adsorbed aluminum gel suspension thus obtained is mixed with a stabilizer and optionally a preservative, and then, the mixture is lyophilized.

The stabilizer is a combination of at least one amino acid or a salt thereof, at least one saccharide and at least one colloidal substance.

Suitable examples of amino acids are glycine, alanine, glutamine, arginine and lysine, or a salt thereof, e.g. monosodium glutamate. They may be used alone or in combination of two or more thereof, and they are usually used in an amount of 0.1 to 2.0 W/V %. Suitable examples of saccharides are monosaccharides such as glucose, xylose, galactose and fructose, disaccharides such as lactose, maltose and saccharose, and sugar alcohols such as mannitol, sorbitol and xylitol, which may be used alone or in combination of two or more thereof. They are usually used in an amount of 0.1 to 15 W/V %. Suitable examples of colloidal substances are gelatin, human albumin and dextrane. They are usually used in an amount of 0 to 0.1 W/V %. Besides, the above-mentioned neutral salt may be added to the solution so as to regulate the content of the neutral salt in the suitable range at this stage.

The thus prepared inactivated, purified HBsAg solution which contains the aluminum gel and a stabilizer is subdivided and filled into vials for each dosage unit package in an amont of 20 μg to 1,000 μg each. The solution in each vial is lyophilized by the conventional rapid lyophilization method or the slow lyophilization method to give the desired lyophilized preparation. The lyophilization is preferably carried out under the following conditions.

The solution is subjected to a pre-lyophilization at a low temperature (e.g. −40°C or lower, preferably −50°C or lower) under atmospheric pressure for several hours (e.g. 3 to 10 hours), and then subjected to a first lyophilization at a fixed higher temperature (e.g. 0° to 8°C) under reduced pressure (e.g. 0.01 to 0.05 Torr) for at least ten hours (e.g. 15 hours), at which stage the temperature of the product becomes lower than −35°C (e.g. about −38°C). Thereafter, the product is subjected to a second lyophilization at a fixed elevated temperature (e.g. 25° to 30°C) under a more reduced pressure (e.g. 0.001 to 0.005 Torr) for several to ten hours (e.g. 6 to 10 hours, preferably 7 to 9 hours).

The lyophilized preparation contains at least HBsAg, a neutral salt, an aluminum gel and a stabilizer.

The lyophilized preparation of hepatitis B vaccine thus obtained has good storage stability and a stable antigen titer. It can be dissolved rapidly in an injection solution when used.

When the lyophilized preparation of the present invention is used, it is dissolved in distilled water for injection or physiological saline solution for injection so as to obtain a HBsAg protein concentration of 5 μg/ml to 40 μg/ml and to obtain an approximately isotonic solution. The physiologically isotonic solution is administered subcutaneously. The dose of the vaccine is usually in the range of 5 μg to 40 μg as HBsAg protein for one administration in adults.

The preparation of the present invention shows no abnormal toxicity when tested in accordance with the conventional test method in guinea pigs as defined by the Minimum Requirements of Biological Products (issued by the Ministry of Health and Welfare, Japan, July 1969). It was also experimentally confirmed by clinical tests, phase II, that the preparation of the present invention is stable and effective.

The present invention is illustrated by the following Examples and Reference Examples but should not be construed to be limited thereto.

Example 1

An inactivated, purified HBsAg is prepared from HBsAg positive human serum by purifying it in a conventional manner, i.e. by density gradient centrifugation using sucrose and a caesium chloride, followed by an inactivation treatment. To a solution of the inactivated, purified HBsAg in a physiological saline solution (HBsAg protein concentration: 128 μg/ml) is added an aqueous solution of aluminum chloride in an amount of 8 times the weight (based on the weight of aluminum hydroxide) of the HBsAg protein. This solution is adjusted to pH 6.7 with 1N NaOH. Aluminum hydroxide gel is produced and simultaneously HBsAg is adsorbed thereon. The HBsAg-adsorbed aluminum gel suspension is divided, and each suspension is centrifuged. After removing the supernatant, to each HBsAg-adsorbed aluminum gel are added aqueous solutions of various kinds of stabilizers and preservatives to give vaccine solutions as shown in Table 1.

1 ml each of these solutions is packed in a 2 ml vial and is subjected to pre-lyophilization at −50°C, under atmospheric pressure for 6 hours, after reducing the pressure to 0.04 Torr, subjected to the first lyophilization at 5°C for 15 hours, and then subjected to the second lyophilization at 30°C, under a pressure of 0.005 Torr for 8 hours, by which the desired lyophilization preparation is obtained.

Table 1

| Run No. | Composition | | | | | | | |
|---|---|---|---|---|---|---|---|---|
| | HBsAg (μg/ml) | Aluminum hydroxide (μg/ml) | NaCl (mg/ml) | Lactose (mg/ml) | Monosodium ℓ-glutamate (mg/ml) | Arginine (mg/ml) | Thimerosal (μg/ml) | Gelatine (mg/ml) |
| 1 | 16 | 128 | 17 | 0 | 0 | 0 | 100 | 0 |
| 2 | 16 | 128 | 10 | 100 | 0 | 0 | 100 | 0 |
| 3 | 16 | 128 | 10 | 100 | 1 | 0 | 100 | 0 |
| 4 | 16 | 128 | 10 | 100 | 2 | 2 | 100 | 0.4 |
| 5 | 16 | 128 | 17 | 20 | 0 | 0 | 100 | 0.4 |

To each of the lyophilized products as obtained above was added distilled water (2 ml), the mixture was shaken for 5 minutes, and then centrifuged. The supernatant was separated, and the amount of free HBsAg in the supernatant was measured by the RPHA (Reverse Passive Hemagglutination) method. The results are shown in Table 2. The RPHA kit used therein had the sensibility of 1 : 128 to a standard solution of HBsAg 1 μg/ml, and each lyophilized sample actually contained 16 μg of HBaAg, i.e., HBsAg with a titer of more than 1 : 1000 in said kit. The supernatant contained HBsAg with a titer of less than 1 : 2 as determined by the RPHA method. This result means that the products almost did not show free HBsAg when dissolved.

## Table 2

| Run No. | RPHA Titer |
|---------|-----------|
| 1 | Less than 1 : 2 |
| 2 | 1 : 2 |
| 3 | Less than 1 : 2 |
| 4 | Less than 1 : 2 |
| 5 | Less than 1 : 2 |

The samples (1 ml each) of Run No. 1 to 5 obtained in the above Example 1 each filled into a 5 ml vial and then subjected to lyophilization treatment in the same manner as above. To the lyophilized products was added a physiological saline solution (each 2 ml) and further added sodium citrate to completely dissolve aluminum gel. HBsAg in the solution was measured by a radioimmunoassay method, as described by Saito et al. in "Application of Parallel Line Bioassay Method to Quantitative Determination of HBsAg in Radioimmunoassay", Japan J, Med. Sci. Biol., 32, 47—52 (1979). In brief, using AUSRIA II (supplied from Dainabbott Radioisotope Lab., Tokyo, Japan), reference for assay was prepared in a concentration of 1 μg/ml HBsAg protein and stored in −80°C after subdivision. The dilution of HBsAg was carried out by employing a dilution buffer of 0.01 M phosphate buffered saline (PBS) containing 1.0 % human serum albumin and 0.1 % polyethylene glycol (PEG 4000). Reference was diluted to 20, 10 and 5 ng/ml HBsAg protein with said dilution buffer, and each Run was also diluted to 20, 10 and 5 ng/ml HBsAg with said dilution buffer. Samples of three aliquots of Reference and each Run were measured in duplicate.

The log 10 net count per minutes (CPM) of each test specimen and the log 10 dilution factor were used for statistical analysis. The parallel line assay method was applied to the validity of assay results and in estimation of relative antigenicity as disclosed in Finney, D.J., Statistical Method in Biological Assay, 2nd Ed., Charles Griffin, London, page 661 (1964).

The data were compared with the results of HBsAg of the products before being lyophilized, on the basis of Reference. The results are shown in Table 3.

## Table 3

| Run No. | Relative antigenicity * |
|---------|-------------------------|
| 1 | 0.21 |
| 2 | 0.69 |
| 3 | 0.74 |
| 4 | 1.00 |
| 5 | 0.79 |

*) Relative value of the antigen of the lyophilized product to that of the product before being lyophilized.

Reference Example 1

Using an inactivated, purified HBsAg, there was prepared a solution of HBsAg (protein concentration: 80 μg/ml), aluminum hydroxide gel (350 μg/ml), sodium chloride (85 mg/ml), thimerosal (50 μg/ml),

formalin (0.005 V/V %) as in the conventional inactivated vaccine. The solution was kept at 37°C and 4°C. When prepared and at each time after having been kept for certain periods, the antigen of the solution was measured by the radioimmunoassay as mentioned before, and the data were compared. Before measurement, to each sample was added sodium citrate buffer to dissolve the aluminum hydroxide gel, by which HBsAg was released from the gel.

The results are shown in Table 4. As is shown in the table, when kept at 4°C, no lowering of antigenicity was observed even after 23 weeks, but when kept at 37°C, it showed rapid lowering of the antigenicity.

Table 4

| Tempera-ture for keeping | Relative antigenicity * | | | | |
|---|---|---|---|---|---|
| | After keeping for: | | | | |
| | 2 weeks | 4 weeks | 8 weeks | 15 weeks | 23 weeks |
| 37°C | 0.57 | 0.22 | 0.16 | 0.19 | 0.00 |
| 4°C | 1.02 | 1.16 | 1.03 | 1.17 | 1.06 |

*) Relative value to antigenicity of the vaccine when prepared.

Example 2

Purified HBsAg, which is prepared from HBsAg positive human serum by ion exchange chromatography, is inactivated in a conventional manner. A solution of the inactivated, purified HBsAg in physiological saline solution (HBsAg protein concentration: 100 µg/ml) is used as vaccine stock solution. To the stock solution (1,600 ml) is added an aqueous solution (80 ml) containing aluminum chloride (800 mg). The solution is adjusted to pH 6.7 with 1N NaOH.

The suspension is centrifuged, and the supernatant is removed. The remaining precipitate is mixed with a physiological saline solution (500 ml) containing lactose (10 W/V %), monosodium l-glutamate (0.4 W/V %), arginine (0.4 W/V %), gelatin (0.08 W/V %) and thimerosal (0.005 W/V %).

The vaccine solution thus obtained is divided and each solution (0.25 ml) is filled into a 2 ml vial, and afterwards subjected to lyophilization treatment in the same manner as described in Example 1.

To the lyophilized products as prepared above was added sodium citrate buffer to dissolve the aluminum gel, and then, the antigen content thereof was measured by radioimmunassay (using AUSRIA-II). The relative titer was calculated based on the titer of the stock solution (as a reference). The results are shown in Table 5.

Table 5

| | Relative titer | 95% Confidence limits |
|---|---|---|
| Reference | 1.0000 | |
| Vaccine | 1.0042 | 0.9097 - 1.11082 |

The lyophilized vacine preparation obtained in Example 2 was compared with the stock solution in terms of immunogenicity in guinea pigs. That is, the lyophilized vaccine preparation was dissolved in distilled water, and the solution was subcutaneously injected in the back of guinea pigs in an innoculation amount of 0.5 µg, 1 µg and 2 µg of HBsAg. After 5 weeks, blood was collected from the animals, and the antibody titer of thus collected blood plasma was measured by the PHA (Passive Hemaggulutination) method. As a reference, the antibody titer in case of innoculation of the stock solution was measured likewise. The results are shown in Table 6.

The average relative potency of the case of innoculation of the lyophilized vaccine to that of the case of innoculation of the stock solution was 4.114. Thus, an adjuvant effect of the aluminum gel was observed.

Table 6

| | Relative potency | 95 % Confidence Limits |
|---|---|---|
| Reference | 1.0000 | |
| Vaccine | 4.1142 | 2.0216 - 17.8621 |

The lyophilized vaccine preparation as obtained in Example 2 was tested for storage stability as follows.

To a sample, which was kept at a fixed temperature for a fixed period of time, was added a sodium citrate buffer to dissolve aluminum gel, and the antigen content thereof was measured by radioimmunoassay. The relative antigenicity was calculated based on HBsAg of the sample when prepared.

The results are shown in Table 7.

As is clear from the results, one year after storage, the preparation was stable at 37°C, which was clearly different from that of a liquid vaccine preparation. Besides, each sample kept at 37°C was subjected to a test for abnormal toxicity. No abnormal toxicity was observed.

Table 7

| Temperature for keeping | Relative antigenicity * | | | | | |
|---|---|---|---|---|---|---|
| | After keeping for: | | | | | |
| | 2 weeks | 4 weeks | 9 weeks | 15 weeks | 23 weeks | 1 year |
| 37°C | 1.14 | 1.10 | 1.16 | 0.92 | 1.11 | 1.14 |
| Room temp. | 1.10 | 0.96 | 1.14 | 1.06 | 1.09 | 1.09 |
| 4°C | 1.16 | 1.09 | 1.03 | 0.98 | 1.10 | 0.99 |

*) Relative value of the antigenicity of the vaccine when prepared.

**Claims for the Contracting States: DE GB FR IT NL CH BE**

1. A lyophilized preparation of hepatitus B vaccine which comprises an inactivated, purified hepatitis B virus surface antigen absorbed on aluminum gel and a stabilizer in the lyophilized state, obtainable by adding an aluminum gel and a stabilizer to an inactivated purified hepatitis B virus surface antigen and lyophilizing the mixture, wherein the stabilizer is a combination of at least one amino acid or a salt thereof, at least on saccharide and at least one colloidal substance.

2. The preparation according to claim 1, wherein the aluminum gel is a member selected from aluminum hydroxide gel and aluminum phosphate gel.

3. The preparation according to claim 2, wherein the amino acid or a salt thereof is a member selected from glycine, alanine, monosodium glutamate, arginine and lysine; the saccharide is a member selected from glucose, xylose, galactose, fructose, lactose, maltose, saccharose, mannitol; sorbital and xylitol; and the colloidal substance is a member selected from gelatin, human albumin and dextrane.

4. A method for the preparation of a lyophilized preparation of hepatitus B vaccine which comprises adding an aluminum gel and a stabilizer to an aqueous solution of an inactivated, purified hepatitis B virus surface antigen wherein the stabilizer is a combination of at least one amino acid or a salt therof, at least one saccharide and at least one colloidal substance, dividing the mixture wherein the hepatitis B virus surface antigen is absorbed onto the aluminum gel into minimum dosage units, and lyophilizing each unit.

5. The method according to claim 4, wherein the lyophilization step comprises three steps consisting of a pre-lyophilization at a low temperature under atmospheric pressure for several hours, a first lyophilization at a higher temperature under reduced pressures for ten to several tens of hours, and a second lyophilization at an elevated temperature under a more reduced pressure for several to ten hours.

**Claims for the Contracting State: AT**

1. A method for the preparation of a lyophilized preparation of hepatitis B vaccine, which comprises adding an aluminum gel and a stabilizer to an aqueous solution of an inactivated, purified hepatitis B virus surface antigen wherein the stablizer is a combination of at least one amino acid or a salt thereof, at least one saccharide and at least one colloidal substance, dividing the mixture wherein the hepatitus B virus surface antigen is absorbed onto the aluminum gel into minimum dosage units, and lyophilizing each unit.

2. The method according to claim 1, wherein the lyophilization step comprises three steps consisting of a pre-lyophilization at a low temperature under atmospheric pressure for several hours, a first lyophilization at a higher temperature under a reduced pressure for ten to several tens of hours, and a second lyophilization at an elevated temperature under a more reduced pressure for several to ten hours.

3. The method according to claim 1, wherein the aluminum gel is a member selected from aluminum hydroxide gel and aluminum phosphate gel.

4. The method according to claim 1, wherein the amino acid or a salt thereof is a member selected from glycine, alanine, monosodium glutamate, arginine and lysine; the saccharide is a member selected from glucose, xylose, galactose, fructose, lactose, maltose, saccharose, mannitol, sorbitol and xylitol; and the colloidal substance is a member selected from gelatin, human albumin and dextrane.

## Patentansprüche für die Vertragsstaaten: BE CH DE FR GB IT NL

1. Gefriergetrocknetes Präparat eines Hepatitis B-Impfstoffes enthaltend ein an Aluminiumgel absorbiertes, inaktiviertes, gereinigtes Hepatitis B-Virus-Oberflächenantigen und einen gefriergetrockneten Stabilisator, erhältlich durch Versetzen eines inaktivierten, gereinigten Hepatitis B-Virus-Oberflächenantigens mit Aluminiumgel und einem Stabilisator, und Gefriertrocknung des Gemisches, wobei der Stabilisator eine Kombination aus mindestens einer Aminosäure oder einem Salz davon, mindestens einem Saccharid und mindestens einer kolloidalen Substanz ist.

2. Präparat nach Anspruch 1, bei dem das Aluminiumgel Aluminiumhydroxidgel oder Aluminiumphosphatgel ist.

3. Präparat nach Anspruch 2, bei dem die Aminosäure oder das Salz davon Glycin, Alanin, Mononatriumglutamat, Arginin oder Lysin ist; das Saccharid Glusoce, Xylose, Galactose, Fructose, Lactose, Maltose, Saccharose, Mannit, Sorbit oder Xylit ist; und die kolloidale Substanz Gelatine, menschliches Albumin oder Dextran ist.

4. Verfahren zur Herstellung eines gefriergetrockneten Präparates eines Hepatitis B-Impfstoffes, bei, dem man eine waßride Lösung eines inaktivierten, gereinigten Hepatitis B-Virus-Oberflächenantigens mit Aluminiumgel und einem Stabilisator versetzt, wobei der Stabilisator eine Kombination aus mindestens einer Aminosäure oder einem Salz davon, mindestens einem Saccharid und mindestens einer kolloidalen Substanz ist, das Gemisch, in dem das Hepatitis B-Virus-Oberflächenantigen an das Aluminiumgel absorbiert ist, in kleinstmögliche Dosierungseinheiten teilt und jede Einheit gefriertrocknet.

5. Verfahren nach Anspruch 4, bei dem der Gefriertrocknungs-Schritt drei Schritte umfaßt, nämlich eine mehrere Stunden dauernde Vorgefriertrocknung bei niedriger Temperatur unter atmosphärischem Druck, eine 10 bis mehrere 10 Stunden dauernde erste Gefriertrocknung bei einer höheren Temperatur unter vermindertem Druck und eine mehrere bis 10 Stunden dauernde zweite Gefriertrocknung bei einer erhöhten Temperatur unter einem noch mehr verminderten Druck.

## Patentansprüche für den Vertragsstaat: AT

1. Verfahren zur Herstellung eines gefriergetrockneten Präparates eines Hepatitis B-Impfstoffes, bei dem man eine waßrige Lösung eine inaktivierten, gereinigten Hepatitis B-Virus-Oberflächenantigens mit Aluminiumgel und einem Stabilisator versetzt, wobei der Stabilisator eine Kombination aus mindestens einer Aminosäure oder einem Salz davon, mindestens einem Saccharid und mindestens einer kolloidalen Substanz ist, das Gemisch, in dem das Hapatitis B-Virus-Oberflächenantigen an das Aluminiumgel absorbiert ist, in kleinstmögliche Dosierungseinheiten teilt und jede Einheit gefriertrocknet.

2. Verfahren nach Anspruch 1, bei dem der Gefriertrocknungs-Schritt drei Schritte umfaßt, nämlich eine mehrere Stunden dauernde Vorgefriertrocknung bei niedriger Temperatur unter atmosphärischem Druck, eine 10 bis mehrere 10 Stunden dauernde erste Gefriertrocknung bei einer höheren Temperatur unter vermindertem Druck und eine mehrere bis 10 Stunden dauernde zweite Gefriertrocknung bei einer erhöhten Temperatur unter einem noch mehr verminderten Druck.

3. Verfahren nach Anspruch 1, bei dem das Aluminiumgel Aluminiumhydroxidgel oder Aluminiumphosphatgel ist.

4. Verfahren nach Anspruch 1, bei dem die Aminosäure oder das Salz davon Glycin, Alanin, Mononatriumglutamat, Arginin oder Lysin ist; das Saccarid Glucose, Xylose, Galactose, Fructose, Lactose, Maltose, Saccharose, Mannit, Sorbit oder Xylit ist; und die kolloidale Substanz Gelatine, menschliches Albumin oder Dextran ist.

## Revendications pour les Etats contractants: DE GB FR IT NL CH BE

1. Une préparation lyopholisée de vaccin d'hépatite B qui comprend un antigène de surface de virus de l'hépatite B purifié, inactivé, absorbé sur un gel d'aluminium et un stabilisant à l'état lyophilisé, pouvant être obtenue par addition d'un gel d'aluminium et d'un stabilisant à un antigène de surface de virus de l'hépatite B purifié, inactivé, et par lyophilisation du mélange, selon laquelle le stabilisant est une combinaison d'au moins un amino-acide ou un sel de celui-ci, d'au moins un saccharide et d'au moins une substance colloïdale.

2. La préparation selon la revendication 1, selon laquelle le gel d'aluminium est choisi parmi un gel d'hydroxyde d'aluminium et un gel de phosphate d'aluminium.

3. La préparation selon la revendication 2, selon laquelle l'aminoacide ou un sel de celui-ci est choisi parmi les suivants: glycine, alanine, glutamate monosodique, arginine et lysine; le saccharide est choisi parmi les suivants: glucose, xylose, galactose, fructose, lactose, maltose, saccharose, mannitol, sorbitol et xylitol; et la substance colloïdale est choisie parmi la gélatine, l'albumine humaine et le dextrane.

4. Un procédé de préparation d'une préparation lyophilisée de vaccin d'hépatite B qui comprend l'addition d'un gel d'aluminium et d'un stabilisant à une solution aqueuse d'un antigène de surface de virus de l'hépatite B purifié, inactivé, dans lequel le stabilisant est une combinaison d'au moins un aminoacide ou un sel de celui-ci, d'au moins un saccharide et d'au moins une substance colloïdale, la division du mélange dans lequel l'antigène de surface de virus de l'hépatite B est absorbé sur le gel d'aluminium en unités de dosage minimales, et la lyophilisation de chaque unité.

5. Le procédé selon la revendication 4, selon lequel l'étape de lyophilisation comprend trois étapes consistant en une prélyophilisation à une basse température à la pression atmosphèrique pendant plusieurs heures, une première lyophilisation à une température supérieure sous une pression réduite pendant 10 h et plusieurs dizaines d'heures, et une seconde lyophilisation à une température élevée sous une pression plus réduite pendant plusieurs heures à 10 h.

**Revendications pour l'Etat contractant: AT**

1. Un procédé de préparation d'une préparation lyophilisée de vaccin d'hépatite B, qui comprend l'addition d'un gel d'aluminium et d'un stabilisant à une solution aqueuse d'un antigène de surface de virus de l'hépatite B purifié, inactivé, selon lequel le stabilisant est une combinaison d'au moins un aminoacide ou un sel de celui-ci, d'au moins un saccharide et d'au moins une substance colloïdale, la division du mélange dans lequel l'antigène de surface de virus de l'hépatite B est absorbé sur le gel d'aluminium en unités de dosage minimales, et la lyophilisation de chaque unité.

2. Le procédé selon la revendication 1, selon lequel l'étape de lyophilisation comprend trois étapes constituées d'une prélyophilisation à une basse température à la pression atmosphérique pendant plusieurs heures, une première lyophilisation à une température supérieure sous une pression réduite pendant 10 h à plusieurs dizaines d'heures, et une seconde lyophilisation à une température élevée sous une pression plus réduite pendant plusieurs heures à 10 h.

3. Le procédé selon la revendication 1, selon lequel le gel d'aluminium est choisi parmi un gel d'hydroxyde d'aluminium, et un gel de phosphate d'aluminium.

4. Le procédé selon la revendication 1, selon lequel l'aminoacide ou un sel de ce dernier est choisi parmi les suivants: glycine, alanine, glutamate monosodique, arginine et lysine; le saccharide est choisi parmi les suivants: glucose, xylose, galactose, fructose, lactose, maltose, saccharose, mannitol, sorbitol et xylitol; et la substance colloïdale est choisie parmi la gélatine, l'albumine humaine et le dextrane.